# EUROPEAN PATENT APPLICATION

(11) **EP 4 574 838 A1**
(43) Date of publication of application: **25.06.2025**
(21) Application number: 23219516.4
(22) Date of filing: 21.12.2023
(51) Int. Cl.: C07K 14/705, A61K 39/00, C12N 15/62

(54) **CHIMERIC ANTIGEN RECEPTOR (CAR) WITH ENHANCED RECRUITMENT OF SIGNALING PARTNERS**

(71) Applicant: Vilnius University, 01513 Vilnius (LT)
(72) Inventor: Arias, Jonathan, Vilnius (LT)
(74) Representative: AAA Law

(57) **Abstract**

The invention relates to a chimeric antigen receptor comprising an antigen-binding domain, wherein said antigen-binding domain comprises a single chain variable fragment; an ectodomain spacer; a synthetic GLUT4 transmembrane peptide composed of 19 to 23 amino acids; and an endodomain. Said chimeric antigen receptor is remarkable in that the synthetic GLUT4 transmembrane peptide yields increased recruitment of signaling partners and tumor clearing properties.

## Description

### FIELD OF THE INVENTION

The present invention relates to a chimeric antigen receptor (CAR) with enhanced recruitment capacity of signaling partners, in particular, DAP10 signaling protein. The activity of this CAR is exemplified by targeting a differentially-expressed marker, which is expressed in leukemia and lymphomas.

### BACKGROUND TO THE INVENTION

Natural killer (NK) cells, hematopoietic stem cell like-cells and induced pluripotent stem cells expressing chimeric antigen receptor (CAR) are useful in the treatment of cancerous diseases. Such cells help to protect the host against viral infections and tumours.

Immune cells such as natural killer, and hematopoietic stem cell-like cells, provide cellular surveillance against oncological diseases. Obtaining and expanding a large number of immune cells specific against an oncological target is often cumbersome but may constitute a therapeutic solution for many cancer types.

Generating a patient-suitable population of immune cells, that is also specific for an oncological target is achieved by the forced expression of CAR protein and the expansion of the cells *ex vivo.* Forced expression of CAR protein on therapeutically relevant cells is typically achieved through transduction of the cells with virus particles. This methodology exposes the therapeutic cells to tumorigenic events, by the risk of integration in unpredictable genomic loci and modifying oncogenes, tumour suppressors or other cellular components. Developing a methodology for expression of CAR without risk of tumorigenic events, and in a defined genomic locus, with a defined genetic dose, remains so far challenging.

CAR proteins are comprised of at least three parts - extracellular ectodomain part, transmembrane part and intracellular endodomain part. The ectodomain part comprises antigen recognition domains such as single chain variable fragments (scFv). The scFv is derived from antibody components, namely heavy chain variable region (VH) and light chain variable region (VL). Fused VH and VL chains compose an scFv. The scFv of CAR are typically fused to a transmembrane peptide through a spacer peptide. In turn, the transmembrane peptide is fused to an intracellular signalling endodomain. When the CAR protein is bound to an antigen target via the scFv, it induces the activation of the cell which expresses it.

Various CAR proteins against tumours have been developed and differ among themselves in their structural components, evoked activity in the cell that expresses it and effectiveness against the target cell. For example, various CAR protein designs are described in WO2015132604A1 and WO2016139487A1. The effectiveness of a CAR can be modulated by various factors which include among other factors: a) the expression level of the CAR protein; b) the nature of the endodomains of the CAR protein; and c) the capacity of CAR protein to recruit signalling partners.

Until now the main therapeutic cell source used to express CAR protein are patient's autologous peripheral blood T-cells. The use of CAR protein on T-cells is defined as CAR-T therapy and the use of NK cells is defined as CAR-NK therapy. As traditionally, mainlyT-cell functionality has been pursued for CAR protein, mainly T-cell specific endodomains have been evaluated.

The selection of the CAR endodomains is traditionally defined by their functional activity in the immune cell intended to express the CAR protein. Other cell type-specific endodomains, apart from T-cell endodomains, and in particular NK cell endodomains have scarcely been evaluated for CAR functionality. Also, cell type-specific transmembrane domains have scarcely been evaluated for CAR functionality in NK cells.

Furthermore, CAR proteins having transmembrane domains with enhanced recruitment of signalling partners have not been developed. The present invention provides a CAR having a synthetic transmembrane domain with enhanced recruitment of signalling partners, in particular, DAP10 signalling protein, resulting in increased functionality.

The endodomains used for CAR until now include the following: CD28, described in EP3241851, CN109468282, US20130071414 and in the study of Savoldo et al. (J. Clin. Invest., 2011, 121(5), 1822-1826); OX40-zeta, described in EP3241851 and in US20130071414; CD3-zeta (CD3z), described in EP3241851; CN109468282; WO2014012001, and in the studies of Milone et al. (Mol. Ther. 2009, 17(8), 1453-1464); Savoldo et al. (cfr. supra); Li et al. (Cell Stem Cell, 2018, 23, 1-12); Lonez et al. (BMJ Open, 2017, 7, e017075); and Grupp et al. (N. Eng. J. Med., 2013, 368, 1509-1518); 4-1BB also called CD137, described in CN109468282, US20050113564, US20130071414 and in the studies of Milone et al. (cfr. supra); and Grupp et al. (cfr. supra); NKR2B4 also called CD244, described in the study of Li et al. (cfr. supra); C3aR, described in CN107903326A; NKG2D, described in the study of Lonez et al. (cfr. supra); and DAP12, described in the study of Töpfer et al., (Front Plant Sci., 2015, 6(49), 1-13).

The transmembrane peptide sequences used for CAR until now include the following: CD8 transmembrane, described in the studies of Grupp et al. (cfr. supra); Milone et al. (cfr. supra); and of Deng Q. et al. (Front Immunol., 2019, 10 (856)); DAP12 transmembrane, described in the study of Töpfer et al. (cfr. supra); NKG2D transmembrane, described in the studies of Li et al. (cfr. Supra) and of Lonez et al. (cfr. supra).

When an immune cell expressing CAR recognizes an antigen-expressing target cell, it induces cell death upon said antigen-expressing target cell.

CD19 is normally expressed at high levels in B-cell malignancies such as Acute Lymphoblastic Leukaemia (ALL). CD19 is an antigen characteristic of early B-cells which is lost at the terminal stage of maturation and differentiation into plasma cells. Moreover, CD19 is not expressed in other cell types of the hematopoietic lineage or non-hematopoietic lineage. Targeting CD19 expressing malignant cells does not lead to toxicity in other tissues or cell types apart from the B-cell tissue.

CAR proteins against tumor antigens are being explored in clinical trials against various tumor types. To date, therapeutic efforts focus mainly on B-cell malignancies.

The present invention concerns a type of chimeric antigen receptor which provides immune activity against a specific oncological target when expressed in immune cells. As an example, the chosen antigen is CD19, as it is an antigen considered a "golden standard" and is used as the proof of concept target to validate new functions of CAR proteins. Here the CD19 target has been used to develop a new CAR with enhanced recruitment capacity of signaling partners. CD19 targeting CAR has used primarily the scFv derived from FMC63 (Nicholson et al., Mol. Immunol., 1997, 34(16-17), 1157-1165).

CAR against CD19 has been described in WO2016139487, WO2014184143, CN109468282, JP6466401, US20130071414, WO2014012001 and in the studies of Grupp et al. (cfr. supra) and Milone et al. (cfr. supra).

US2019/0062430 describes CAR-T cells modified to express a CAR fusion protein comprising from N-terminus to C-terminus a scFv with an activity against CD19 and comprising VH and VL, a transmembrane domain, at least one co-stimulatory domain and an activating domain. The modified CAR-T cell also comprises a FLAG-tag, which helps to reduce cytokine levels. The transmembrane domain comprises 27 amino acids.

US2019/0106492 describes CAR containing human CD19 antigen binding domains. It discloses more specifically a CD8 transmembrane made of 22 amino acids that is located between the hinge (spacer) region and the signaling domain of the CAR.

WO2018/226958 describes CAR containing both CD19 and CD79 antigen binding domains. The transmembrane peptide of this CAR comprises amino acids which stays uncharged at physiological pH, i.e. at a pH of 7.4.

WO2019/126639 describes CAR containing the CD19 tumor antigen. The CAR further comprises a human transferrin agent. It is more specifically disclosed that the transmembrane peptide domain comprises hydrophobic residues such as leucine and valine. The transmembrane peptide has also a length of at least 24 amino acids. Transmembrane peptide with hydrophobic residues is also described in WO 2017/216562 and in US 2018/0142034.

WO2018/195339 provides an immune cell engineered to express human interleukin-15 and at least two antigen receptors that can be a CAR and/or a T-cell receptor. The tumor associated antigen can be CD19 among others. It is disclosed that the transmembrane domain is positively charged.

US11020429B2 describes recombinant molecules expressed in cells which are or interact with GLUT4. It further discloses cells which generally further express an immune receptor, such as an antigen receptor, which may be an engineered receptor, such as a CAR or recombinant TCR. US11655452B2 describes method of reducing the number of target cells. It further discloses that cells are targeted based on the expression of CD68.

US11130820B2 described a tumor-associated antigen or a tumor-specific antigen recognizing CAR. It further discloses that the antigen recognized is a CD68\KP1.

Obtaining sufficient immune cells from a human source and even more from a person with a compromised immune system, such as one suffering cancer is generally troublesome and unsafe for the patient's health.

Traditionally, obtaining immune cells for the generation of CAR containing cells is achieved by leukapheresis of patients own blood. This approach imposes two risks: a) transient removal of tumor-specific immune cells from the patient's system and b) co-purification of tumor cells along with the intended immune cells. Traditionally, then the purified immune cells are transduced with virus particles containing CAR.

The use of patient own immune cell poses the risk of co-purifying cancer cells and engineering CAR onto said cancer cells. Recent reports confirm this phenomenon occurs (Ruella et al., Nat. Med., 2018, 24(10), 1499-1503). This poses a high risk to all ongoing clinical trials and constitutes a major challenge in CAR technology.

Modulating the capacity of a CAR protein to recruit signalling partners remains so far challenging. The present invention addresses these factors and presents a CAR with improved capacity to recruit DAP10 signalling protein.

### SUMMARY OF THE INVENTION

In a first aspect, the invention provides a chimeric antigen receptor, alias CAR, comprising
a. an antigen-binding domain, wherein said antigen-binding domain is a differentially expressed marker-binding domain, for example, a CD19-binding domain, and comprises a single chain variable fragment, alias scFv;
b. an ectodomain spacer, for example, wherein said spacer domain encompasses a CD68 peptide;
c. a synthetic GLUT4 transmembrane peptide composed of 19 to 23 amino acids, wherein said GLUT4 transmembrane comprises at least one amino acid selected among arginine, histidine or lysine;
d. endodomain, for example, a GLUT4 endodomain, preferably wherein the GLUT4 endodomain encompasses at least one di-leucine motif,
wherein in the at least one of the amino acids from 1 to 13 of said synthetic GLUT4 transmembrane peptide is selected from arginine, histidine or lysine.

Said chimeric antigen receptor is remarkable in that the synthetic GLUT4 transmembrane confers enhanced recruitment of signalling partners.

With preference, the synthetic GLUT4 transmembrane peptide comprises among the amino acids 1 to 13 at least one arginine. In some other embodiments, the synthetic GLUT4 transmembrane peptide comprises among the amino acids 1 to 13 at least two arginines.

Surprisingly, the inventors have found that genetic engineering with integrating vectors affords to express one or a plurality of antigen specific CAR in cells of non-immune type, such as induced pluripotent stem cells (iPSC) and patient-derived iPSC, which in turn can be differentiated into sufficient immune cells such as hematopoietic stem cell-like cells, T cell or iPSC-derived natural killer cells, mainly iPSC-derived natural killer cells. As the amino acid 1 to 13 on the N-terminus of the synthetic transmembrane peptide comprises one or more positive charges, it allows for the enhancement of the interaction, and the interaction and recruitment of DAP10 signaling protein. Since DAP10 functions as an adaptor protein (i.e. a protein that facilitates the creation of signaling complexes) by recruiting the p85 subunit of phosphoinositide 3-kinase (PI3K), the activation of PI3K is revealed. PI3K is a family of an enzyme involved in cellular functions such as cell growth, proliferation, differentiation, motility, survival, and intracellular trafficking, which in turn, are involved in cancer. This allows for obtaining sufficient and a large number of oncological target-specific CAR expressing immune cells, containing a CAR with enhanced ability to recruit DAP10 signaling partner. The present invention yields a CAR with enhanced binding of signaling partners, which is expressed from a defined genomic locus with defined genetic dose.

With preference, one or more of the following features can be used to better define the single chain variable fragment (scFv) of the antigen-binding domain of the CAR of the present invention:
- a heavy chain variable region (VH); and/or
- a light chain variable region (VL).
- Said VL and VH of said scFv are connected to each other with a linker, which has, for example, a sequence as indicated in SEQ ID No. 1
- Said scFv of said antigen-binding domain is oriented in the direction VH-VL or VL-VH.

With preference, the scFv of said CD19-binding domain of the antigen-binding domain of the CAR of the present invention, wherein said fragment is composed of:
- a heavy chain variable region (VH), for example, with a sequence of VH as indicated in SEQ ID No. 2; and/or
- a light chain variable region (VL), for example, with a sequence as indicated in SEQ ID No. 3.
- Said scFv of said antigen-binding domain is oriented in the direction VH-VL or VL-VH, for example, with one of the following sequences indicated in any one of SEQ ID No. 4 to 5.

With preference, one or more of the following features can be used to better define the ectodomain spacer of the CAR of the present invention:
- Said ectodomain spacer comprises at least one CD68 peptide.
- The CD68 ectodomain spacer peptide comprises a sequence selected from SEQ ID No. 7, SEQ ID No. 8, SEQ ID No. 9, SEQ ID No. 10, SEQ ID No. 11, or SEQ ID No. 12, or a variant thereof having at least 80% sequence identity; more preferably one of SEQ ID No. 7 or SEQ ID No. 12, or a variant thereof having at least 80% sequence identity, even more preferably said ectodomain spacer peptide comprises SEQ ID No. 12 or a variant thereof having at least 80% of sequence identity.

With preference, one or more of the following features can be used to better define the synthetic GLUT4 transmembrane peptide of the CAR of the present invention:
- the synthetic GLUT4 transmembrane peptide comprises one of SEQ ID No. 13, SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 29, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, or SEQ ID No. 45, or a variant thereof having at least 80% sequence identity; more preferably one of SEQ ID No. 38 or SEQ ID No. 39, or a variant thereof having at least 80% sequence identity, even more preferably said synthetic transmembrane peptide comprises SEQ ID No. 39 or a variant thereof having at least 80% of sequence identity.

With preference, one or more of the following features can be used to better define the GLUT4 endodomain of the CAR of the present invention:
- The GLUT4 endodomain comprises one of SEQ ID No. 46, SEQ ID No. 47, or SEQ ID No. 48, or a variant thereof having at least 80% sequence identity; more preferably one of SEQ ID No. 48, or a variant thereof having at least 80% sequence identity.
- The GLUT4 endodomain comprises a di-leucine motif.

With preference, said CAR comprises one of the following sequences as indicated in SEQ ID No. 49, SEQ ID No. 50, SEQ ID No. 51, SEQ ID No. 52, SEQ ID No. 53, SEQ ID No. 54, SEQ ID No. 55, SEQ ID No. 56, SEQ ID No. 57, SEQ ID No. 58, SEQ ID No. 59, SEQ ID No. 60, SEQ ID No. 61, SEQ ID No. 62, SEQ ID No. 63, or SEQ ID No. 64, or a variant thereof having at least 80% sequence identity; more preferably one of the following sequences as indicated in SEQ ID No. 55, SEQ ID No. 56, SEQ ID No. 63, or SEQ ID No. 64, or a variant thereof having at least 80% of sequence identity, even more preferably the sequence indicated in SEQ ID No. 63, or SEQ ID No. 64, or a variant thereof having at least 80% of sequence identity, even most preferably the sequence indicated in SEQ ID No. 64, or a variant thereof having at least 80% of sequence identity.

With preference, the CAR comprises a signal peptide advantageously fused to the N-terminus of the single chain variable fragment.

In a second aspect, the invention provides for a nucleic acid sequence which encodes a CAR according to the first aspect.

In a third aspect, the invention provides for a vector which comprises a nucleic acid sequence according to the second aspect of the invention.

In a fourth aspect, the invention provides for a cell which comprises a CAR according to the first aspect of the invention and/or a nucleic acid sequence according to the second aspect of the invention.

With preference, said cell is a natural killer cell, an induced natural killer cell, a hematopoietic stem cell, a hematopoietic stem cell like-cell, an induced pluripotent stem cell, or an induced pluripotent stem cells-derived natural killer cell; more preferably an induced pluripotent stem cells-derived natural killer cell.

In a fifth aspect, the invention provides for a pharmaceutical composition which comprises a cell according to the fourth aspect of the invention together with a pharmaceutically acceptable carrier, diluent and/or excipient.

In a sixth aspect, the invention provides for a cell in accordance with the fourth aspect of the invention or a pharmaceutical composition in accordance with the fifth aspect of the invention for use in treating cancer; with preference, for use in a treatment of CD19 positive B cell malignancy.

In a seventh aspect, the invention provides for a method for making a cell according to the fourth aspect of the invention, which comprises the step of transducing or transfecting a cell with a vector according to the third aspect of the invention.

With preference, said step of transducing or transfecting a cell with said vector is performed ex *vivo.*

### DESCRIPTION OF THE FIGURES

Figure 1: Quantification of DAP10 co-immunoprecipitated from HYG1 cells expressing CAR protein of SEQ ID No. 57 to SEQ ID No. 64 fused with MYC tag.
Figure 2: Quantification of PI3K and AKT pathway activity in HYG1 cells expressing CAR protein of SEQ ID No. 57 to SEQ ID No. 64 and a FOXO-luciferase reporter.
Figure 3: Quantification of cell death of malignant cells by release of LDH (lactate dehydrogenase) protein when co-culture of HYG1 cells expressing CAR protein of SEQ ID No. 57 to SEQ ID No. 64 and CD19 expressing malignant cells SU-DHL-4.
Figure 4: Quantification of genetic dose of CAR genes containing SEQ ID No. 57 to SEQ ID No. 64 integrated into the genome of iPSC clones.
Figure 5: Quantification of DAP10 co-immunoprecipitated from NK cells derived from iPSC (iPSC-derived NK cells), expressing CAR protein of SEQ ID No. 57 to SEQ ID No. 64 fused with MYC tag.
Figure 6: Quantification of PI3K and AKT pathway activity in NK cells derived from iPSC (iPSC-derived NK cells) expressing CAR protein of SEQ ID No. 57 to SEQ ID No. 64 and a FOXO-luciferase reporter.
Figure 7: Quantification of cell death of malignant cells by release of LDH (lactate dehydrogenase) protein. Co-culture of NK cells derived from iPSC (iPSC-derived NK cells) expressing CAR protein of SEQ ID No. 57 to SEQ ID No. 64 and CD19 expressing malignant cells SU-DHL-4.

### DETAILED DESCRIPTION

The present invention relates to a new CD19-specific CAR which evokes in the CAR expressing-cells an enhanced recruitment of signalling partners.

### CHIMERIC ANTIGEN RECEPTORS (CARs)

Chimeric antigen receptors (CARs) provide a predetermined specificity to the cell that expresses them and codes specific activation pathways in said cells. Traditionally, CARs are expressed in immune effector cells. CARs can be expressed in non-immune cells which in turn can then be differentiated into immune effector cells. The most frequently used immune effector cell is a T-cell, and the predetermined antigen binding CAR is expressed in a T-cell. CAR protein can provide therapeutic properties to other immune effector cells such as natural killer cells. CAR-encoding nucleic acids are traditionally transferred to a cell using viral particles. A CAR can be transferred to a cell using precise integration methods which are unaffected by the tumorigenic risk of viral particles. In turn, sufficient patient-suitable and cancer-specific cells can be generated for adoptive cell transfer. Phase II clinical studies of this approach show efficacy.

The target-antigen binding domain of a CAR is commonly fused via a spacer and transmembrane domain to an endodomain. The transmembrane domain or endodomain may associate with an NK cell signalling domain. The transmembrane domain or endodomain may recruit NK cell signalling domains. When the CAR binds the target antigen, this results in the transmission of an activating signal to the cell that expresses it.

### DIFFERENTIALLY EXPRESSED MARKER-BINDING DOMAIN

The CAR of the present invention comprises an antigen-binding domain (for example, a CD19-binding domain) which is composed of a single chain variable region (scFv). The scFv is a fusion protein, which comprises a heavy chain variable region (VH) and a light chain variable region (VL), connected by a peptide linker of up to 30 amino acids. The VH and VL placement is in the order VH-VL, with VH in the amino terminus of the CAR protein, and the VL is connected to the carboxy terminus of VH through a linker. The scFv is connected to the amino terminus of an ectodomain spacer. The ectodomain spacer (for example, the CD68 ectodomain) is connected to the amino terminus of a synthetic GLUT4 transmembrane domain, which is connected to the amino terminus of the endodomain (for example, a GLUT4 endodomain). That means that structurally, the amino-terminus of the endodomains is closer to the membrane than its carboxy-terminus.

The single chain variable region (scFv) of the present invention comprises a heavy chain variable region (VH), with the following sequences:
With preference, the sequence VH indicated in any one of SEQ ID No. 2.

In addition, the single chain variable region (scFv) of the present invention comprises a light chain variable region (VL), with the following sequences:
With preference, the sequence VL indicated in any one of SEQ ID No. 3.

It may be possible to introduce one or more mutations, including substitutions, additions or deletions, into one or more scFv to improve the antigen-binding activity (for example, to improve CD19-binding activity). One or more variable region could, for example, have one, two or three amino acid mutations. The one or more variable regions may be grafted to humanized scFv.

For example, the CAR of the present invention may comprise a CD19-binding domain consisting or comprising one of the following sequences: SEQ ID No: 1, SEQ ID No: 2, SEQ ID No: 3, SEQ ID No: 4, SEQ ID No: 5.

In one embodiment, the scFv binding domain can be made of three segments, indicated respectively in SEQ ID No. 1, SEQ ID No. 2, SEQ ID No. 3, wherein the C-terminus of SEQ ID No. 2 or SEQ ID No. 3, is attached to the N-terminus of the SEQ ID No. 1, wherein the C-terminus of the one of SEQ ID No. 1 is attached to the N-terminus of SEQ ID No. 2 or SEQ ID No. 3.

With preference, the CAR of the invention may comprise a variant of the sequences indicated in any one of the SEQ ID No.1 to 5 with at least 80% of sequence identity, provided that the variant sequence retains the capacity to bind a differentially expressed marker through a composed VH and VL binding, more preferentially with at least 85%, even more preferentially with at least 90%, most preferentially with at least 95%, even most preferentially with at least 98%, even more advantageously with at least 99% of sequence identity.

The percentage identity between two polypeptide sequences may be readily determined by programs such as Clustal Omega, which is freely available at https://www.ebi.ac.uk/Tools/msa/clustalo/

### ECTODOMAIN SPACER

The CAR of the invention further comprises an ectodomain spacer. Advantageously, the CAR of the invention may comprise a synthetic CD68 ectodomain spacer protein. It may comprise a globular and soluble domain with beta-sheets and loops with or without cysteine amino acids positioned along the globular domain. The ectodomain spacer may be derived from CD68. The ectodomain spacer may comprise one of the sequences indicated as SEQ ID No. 6, which is a sequence from human origin, or indicated as SEQ ID No. 7, SEQ ID No. 8, SEQ ID No. 9, SEQ ID No. 10, SEQ ID No. 11, or SEQ ID No. 12. The SEQ ID No. 7 to 12 are artificial sequences. Advantageously, SEQ ID No. 7 and SEQ ID No. 12, in particular SEQ ID No. 12, when incorporated into a CAR protein, provide the best results in term of recruitment of DAP10 signalling partners.

### TRANSMEMBRANE DOMAIN

The CAR of the invention advantageously further comprises a synthetic GLUT4 transmembrane peptide. It may comprise a hydrophobic alpha helix with positively charged amino acids positioned along the helix to recruit and interact with other transmembrane proteins. The transmembrane domain may be derived from GLUT4. The transmembrane domain may comprise one of the sequences indicated as SEQ ID No. 13, which is a sequence from human origin, or indicated as SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 29, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, or SEQ ID No. 45. The SEQ ID No. 14 to 45 are artificial sequences. Preferably, the transmembrane domain comprises one of the sequences indicated as SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, or SEQ ID No. 45. Advantageously, SEQ ID No. 38 and SEQ ID No. 39, in particular SEQ ID No. 39, when incorporated into a CAR protein, provide the best results in term of recruitment of DAP10 signalling partners.

The sequences indicated in SEQ ID No. 13 to SEQ ID No. 45 comprise 23 amino acids in length and are flanked by alanine at the N-terminus and by Leucine in the C-terminus.

The present invention found that the synthetic transmembrane domain presents an enhanced capacity to recruit signalling partners, including DAP10, compared to CAR containing native counterparts. The transmembrane domain may comprise the SEQ ID No. 13 to 45 or a variant thereof having 5, 4, 3, 2 or 1 amino acid mutations (insertions, substitutions or additions) provided that the transmembrane domain still functions to cause enhanced recruitment of signalling partners in the cell expressing the CAR. For example, the transmembrane domain may comprise variants the SEQ ID No. 13 to 45, wherein the variants comprise from 19 to 23 amino acids (i.e. 19, 20, 21, 22 or 23 amino acids), provided that the transmembrane domain still functions to cause enhanced recruitment of signalling partners in the cell expressing the CAR. The presence of a positive charge on the first half of the transmembrane domain is important in order to cause an enhanced recruitment of signalling partners in the cell expressing the CAR. In particular, the presence of a positively charged amino acids, such as arginine, histidine or lysine, in the amino acids 1 to 13 of the transmembrane domain is advantageous in providing the necessary interactions with signalling partners such as DAP10.

### ENDODOMAIN - INTRACELLULAR CELL SIGNALING DOMAIN

The endodomain is the signal-transmission portion of the CAR. After antigen recognition, receptors cluster and a signal are transmitted to the cell. The most commonly used endodomain component is that of CD3z. This transmits an activation signal to the T-cell after antigen is bound. CD3z may not provide a fully competent activation signal and additional co-stimulatory signaling may be needed. For example, endodomains from NKR2B4 and NKG2DS can be used with CD3z to transmit a proliferative or survival signal, or all three can be used together.

Earlier, first-generation CAR T-cells contained only a CD3z endodomain, which recapitulates "signal 1" of T cell activation. First-generation CAR T-cells evoked poor antitumor efficacy in patients, due to the limited expansion capacity and persistence of transferred T-cells. Compound endodomains were created to overcome the limitations in expansion and persistence.

The second-generation CAR added intracellular signaling domains from various costimulatory molecules such as CD28, 4-1BB and OX40 to the first-generation CAR, which improved the proliferation, cytotoxicity, sustained response, and life span of CAR-T cells *in vivo.*

In the third-generation CAR, two costimulatory molecules were fused to the CD3z signaling moiety, with the most common combinations being of p56-LCK CD28 CD3z, OX40 CD28 CD3z, or 4-1BB CD28 CD3z. The third-generation CAR can reduce the undesired anti-inflammatory effect of IL-10, but takes the risk of signal leakage and cytokine cascade.

To optimize the anti-tumor effects of CAR T cells, the fourth generation CAR has been developed by engineering the second-generation CAR with a cytokine expression cassette, which is known as T-cells redirected for universal cytokine-mediated killing (TRUCK). TRUCK can strengthen T-cell activation and attract innate immune cells to the targeted lesion to eradicate antigen-negative tumor cells by releasing anti-tumor cytokines, thus producing better anti-tumor effects, especially on solid tumors.

CAR of the present invention comprises an endodomain, for example any endodomain as described above. With preference, the endodomain or endodomains of the CAR of the present invention consist of synthetic fusion proteins which may comprise combinations of one or more of the following endodomains indicated in SEQ ID No. 46 (GLUT4 endodomain from human origin), SEQ ID No. 47 (GLUT4 endodomain from human origin), or SEQ ID No. 48 (synthetic endodomain, which is the fusion between a GLUT4 endodomain as indicated in SEQ ID No. 47 and a GLUT4 endodomain as indicated in SEQ ID No. 46).

More preferably, the endodomains of the CAR of the present invention comprises at least one endodomain as indicated in SEQ ID No. 46, or in SEQ ID No. 48. Even more preferably, the endodomains of the CAR of the present invention comprises at least one endodomain as indicated in SEQ ID No. 48 with a di-leucine motif.

With preference, the intracellular signaling domain (endodomain) of the CAR of the present invention may comprise one or more of the sequences indicated as SEQ ID No. 46 to 48 or a variant thereof having at least 80% of sequence identity, more preferably at least 85%, even more preferably at least 90%, most preferably at least 95%, even most preferably at least 98%, even more advantageously with at least 99% of sequence identity provided that the sequence provides an effective transmembrane domain or intracellular NK cell signaling domain.

### SIGNAL PEPTIDE

The CAR of the present invention may also comprise a signal peptide so that when the CAR is expressed inside a cell, such as a NK cell, the nascent protein is directed to the endoplasmic reticulum and subsequently to the cell surface, where it is expressed. The core of the signal peptide may contain a long stretch of hydrophobic amino acids that has a tendency to form a single alpha-helix. The signal peptide may begin with a short positively charged stretch of amino acids, which helps to enforce proper topology of the polypeptide during translocation. At the end of the signal peptide, there is typically a stretch of amino acids that is recognized and cleaved by signal peptidase. Signal peptidase may cleave either during or after completion of translocation to generate a free signal peptide and a mature protein. The free signal peptides are then digested by specific proteases. The signal peptide may be at the amino terminus of the molecule, preferably at the N-terminus of the CAR.

The signal peptide may comprise a CD8B peptide as indicated in SEQ ID No. 65 from human origin or a variant thereof having 5, 4, 3, 2 or 1 amino acid mutations (insertions, substitutions or additions) provided that the signal peptide still functions to cause cell surface expression of the CAR.

### RECRUITMENT OF SIGNALING PARTNERS

The present inventors have found that a CAR that has properties which result in enhanced recruitment of signalling partners including DAP10 signaling protein.

Given that the main experience with CD19 CAR therapy has been with CARs based on the fmc63 scFv, and that the oldest, largest and perhaps most significant clinical data set is with the fmc63 based Campana CAR, the present invention took this Campana CAR as the proof of concept targeting domain.

Given that mainly T-cell based transmembrane domains has been used as NK-cell specific transmembrane domains, the present inventors tested novel transmembrane domains for their properties on NK cells, particularly transmembrane domains expressed in muscle tissue, particularly GLUT4 transmembrane domains.

Surprisingly, the present inventors found that while the CAR proteins with the synthetic GLUT4 transmembrane domains described in the present invention provide transmembrane linking properties, in addition, they also provide enhanced recruitment of signalling partners, in particular, DAP10.

Further, a tumour clearing assay of an aggressive B-cell lymphoma showed improved clearing capacity of the cells expressing the synthetic GLUT4 transmembrane domains with enhanced recruitment capacity of signalling partners.

The CAR of the invention may cause 25, 50, 75 or 90% faster clearing of CD19 positive tumour cells in contact with CAR expressing NK cells.

The CAR of the invention may result in a larger proportion of CAR NK cells becoming activated by the CD19 expressing malignant cells. NK cell activation may be assessed using methods known in the art, such as analysis of phosphoinositide 3-kinase (PI3K) activation. The CAR of the present invention may cause 20, 30, 40, 50, 60 of 70% higher activation CAR NK cells to activate the PI3K pathway in a comparative assay involving bringing CAR NK cells into contact with CD19 positive target malignant cells.

### CAR EXAMPLES

The CAR of the invention may have the general formula, wherein all the proteins domains as detailed above are fused together according to the following order (from N-terminus to C-terminus):
antigen-binding domain - ectodomain spacer domain - GLUT4 transmembrane domain - endodomain signalling domain(s).

In preferred embodiments, the protein domains are fused as follows: antigen-binding domain - CD68 ectodomain spacer domain - GLUT4 transmembrane domain - GLUT4 endodomain signalling domain(s).

Subsequently, the CAR with enhanced capacity to recruit signalling partners may include any one of the sequences indicated in SEQ ID No. 49, SEQ ID No. 50, SEQ ID No. 51, SEQ ID No. 52, SEQ ID No. 53, SEQ ID No. 54, SEQ ID No. 55, SEQ ID No. 56, SEQ ID No. 57, SEQ ID No. 58, SEQ ID No. 59, SEQ ID No. 60, SEQ ID No. 61, SEQ ID No. 62, SEQ ID No. 63 or SEQ ID No. 64, or a variant thereof having at least 80% sequence identity.

Advantageously, SEQ ID No. 63 and SEQ ID No. 64, in particular SEQ ID No. 64, provide the best results in term of recruitment of DAP10 signalling partners.

If a variant of one of these sequences is selected, such variant has preferably at least 80% sequence identity, more preferably at least 85% sequence identity, even more preferably at least 90% sequence identity, most preferably at least 95% sequence identity or even most preferably at least 99% sequence identity.

In some cases, the CAR with enhanced capacity to recruit signalling partners may include an amino acids sequence portion starting from position 27 of any one of the sequences indicated in SEQ ID No. 49, SEQ ID No. 50, SEQ ID No. 51, SEQ ID No. 52, SEQ ID No. 53, SEQ ID No. 54, SEQ ID No. 55, SEQ ID No. 56, SEQ ID No. 57, SEQ ID No. 58, SEQ ID No. 59, SEQ ID No. 60, SEQ ID No. 61, SEQ ID No. 62, SEQ ID No. 63 or SEQ ID No. 64, or a variant thereof having at least 80% sequence identity. In some cases, the CAR with enhanced capacity to recruit signalling partners may include an amino acids sequence portion starting from position 39 of any one of the sequences indicated in SEQ ID No. 49, SEQ ID No. 50, SEQ ID No. 51, SEQ ID No. 52, SEQ ID No. 53, SEQ ID No. 54, SEQ ID No. 55, SEQ ID No. 56, SEQ ID No. 57, SEQ ID No. 58, SEQ ID No. 59, SEQ ID No. 60, SEQ ID No. 61, SEQ ID No. 62, SEQ ID No. 63 or SEQ ID No. 64, or a variant thereof having at least 80% sequence identity.

### NUCLEIC ACID SEQUENCE

The second aspect of the invention relates to a nucleic acid sequence which codes for a CAR as described above.

With preference, said nucleic acid sequence comprises one or more of the following features:
- Elements for its integration into the human genome and/or its persistence as an episome (namely a molecule of circular DNA, said molecules capable of autonomous reproduction and also integration within the genome of the host cell), said elements being preferentially selected from one or more of the following: homology arms, long terminal repeats, internal-terminal repeats, recombinase binding sites, terminal repeats, direct repeats, inverted repeats, palindromic repeats, transposon binding sites, EBNA coding sequence, Dyad symmetry, family of repeats and/or OriP.
- Homology arm sequences homologous to the nucleotide sequences of a human cell which allows homologous recombination with the nucleotide sequences comprised in a human cell.

The DNA sequences SEQ ID No. 66 to SEQ ID No. 73 are the nucleic acid sequences encoding respectively for the protein CAR sequences SEQ ID No. 57 to SEQ ID No. 64.

### VECTOR

The present invention also provides a vector which comprises a nucleic acid sequence as described above. Such a vector may be used to introduce the nucleic acid sequence into a host cell so that it expresses and produces a molecule according to the first aspect of the invention. The vector may, for example, be a plasmid or an episome or a viral vector, such as an homology directed repair donor vector, an episomal vector, a retroviral vector, an AAV vector or a lentiviral vector. The vector may be capable of recombining, being retained extrachromosomally, transfecting or transducing a cell, such as a natural killer (NK) cell or an induced pluripotent stem cells (iPSC)-derived natural killer cell, preferably an iPSC-derived natural killer cell.

### CELL

The invention also provides a cell which comprises a nucleic acid according to the invention. The invention provides a cell which expresses a CAR as described above at the cell surface. The cell may be a cytolytic immune cell, such as NK cell or iPSC-derived natural killer cell, preferably an iPSC-derived natural killer cell.

A cell capable of expressing a CAR according to the invention may be made by recombining, episome retaining, transducing or transfecting a cell with CAR-encoding nucleic acid. The CAR-expressing cell of the invention may be generated ex vivo. The cell may be from a cell sample, such as fibroblasts from a skin biopsy or peripheral blood mononuclear cell (PBMC) sample from the patient or a donor. Cells may be reprogrammed prior to incorporating CAR-encoding nucleic acids, for example by treatment with stem cell transcriptional factors such as KLF4, SOX2, NANOG, LMYC, POU5F1 and TP53 null. Cells may be differentiated to immune effector cells before or after incorporating CAR-encoding nucleic acids, for example by treatment with cytokines, growth factors, small molecules or extracellular attachment molecules.

### PHARMACEUTICAL COMPOSITION

The present invention also relates to a pharmaceutical composition containing a CAR-expressing cell, or a plurality of cells, of the invention together with a pharmaceutically acceptable carrier, diluent or excipient, and optionally one or more pharmaceutically active polypeptides and/or pharmaceutically active compounds. Such a formulation may, for example, be in a form suitable for intravenous infusion.

### METHOD OF TREATMENT

CAR-expressing cells of the present invention may be capable of killing cancer cells, such as B-cell lymphoma cells. CAR-expressing cells, such as NK cells or iPSC-derived natural killer cells, preferably iPSC-derived natural killer cells, may either be created ex vivo either from a patient's own fibroblasts or peripheral blood (1st party), or from a connected donor's fibroblasts or peripheral blood (2nd party), or from an unconnected donor's fibroblasts or peripheral blood (3rd party). Alternatively, CAR-expressing cells may be derived from ex vivo differentiation of inducible progenitor cells or induced pluripotent stem cells to cells such as NK cells. In these instances, CAR-containing cells are generated by introducing DNA or RNA coding for the CAR by one of many means including transfection with DNA or RNA, or transduction with a viral vector. NK cells or iPSC-derived NK cells expressing a CAR molecule of the present invention may be used for the treatment of a cancerous disease, in particular, a cancerous disease associated with CD19 expression.

A method for the treatment of disease relates to the therapeutic use of a cell or population of cells of the invention. In this respect, the cells may be administered to a subject having an existing disease or condition in order to lessen, reduce or improve at least one symptom associated with the disease and or to slow down, reduce or block the progression of the disease. The method of the invention may cause or promote cell mediated killing of CD19-expressing cells, such as malignant B cells.

### SYNTHESIS OF THE SEQUENCES

A desired CAR protein sequence is defined *in silica,* then the DNA sequence that codes said desired CAR protein sequence is also defined in silico. The DNA sequence that codes said desired protein constitutes an open reading frame (ORF), it starts with a start-codon (methionine) and ends with a termination codon (TGA, TAA or TAG), and that ORF constitutes a synthetic CAR gene. In turn, the synthetic CAR gene is codon-optimized for human cells. DNA sequences are synthesized in arbitrary fragments of overlapping single stranded oligo nucleotides as in the work of Cha-aim et al. (Gene synthesis: Methods and Protocols, 2012, 852, 97-110).

Single stranded oligo nucleotides act as both primers and templates in an assembly polymerase chain reaction (PCR). This type of PCR is called fusion-PCR. Alternatively, complementary or partially complementary single stranded oligo nucleotides are hybridized to create double stranded DNA, which in turn is cloned into compatible vectors. Alternatively, double stranded DNA is cloned using endonucleases and DNA ligases into compatible vectors. Alternatively, double stranded DNA molecules with overlapping homology are fused by using an enzyme containing 3' exonuclease or 5' exonuclease activity assisted by the complementary annealing properties of the DNA molecule. Alternatively, interruptions in the DNA strands can be displaced using DNA polymerases. Alternatively, nicks present in the DNA strands can be ligated using DNA ligases.

The desired synthetic CAR gene sequences are then cloned under the control of a promoter located in its 5' sequence. The synthetic CAR gene is downstream of and controlled by a ribosome binding site. The synthetic CAR gene is flanked at its 3' by a polyadenylation signal. The synthetic CAR gene may contain additional regulatory elements in its 5' untranslated region or 3' untranslated region, such as promoters, transcription factor binding sites, enhancers, introns, exons, microRNA binding sites or internal ribosome binding sites. All the cis acting elements required for the transcription and translation of the synthetic CAR gene can be contained in the resulting DNA molecule, gene and vector.

When the synthetic CAR gene is contained within a cell's genome or episome, the cell expresses the messenger RNA (mRNA) of said synthetic CAR gene and translates that mRNA into the CAR protein. The CAR protein expressed in that cell, in turn, exerts its binding, interacting and signalling functions, conferring therapeutic properties to the cell that contains it. Transcription and translation of the synthetic CAR gene can thus take place in vivo.

Alternatively to the in vivo transcription of the synthetic CAR gene, the synthetic CAR gene can be transcribed to mRNA molecules in vitro. In vitro mRNA transcription can be performed using an RNA polymerase, such as T7 RNA polymerase. The mRNA molecule can then be polyadenylated in vitro using a poly-A polymerase, such as poly-A polymerase I. Alternatively to the in vivo translation of the synthetic CAR gene transcripts, CAR mRNA molecules can be used as substrate for protein translation in vitro. In vitro translation of CAR mRNA molecules can be performed using synthetic mixtures of 70S or 80S ribosomes, tRNAs, aminoacyl-tRNA synthetases, initiation, elongation and termination factors, amino acids, ATP, GTP, creatine phosphate, creatine phosphokinase, phosphoenol pyruvate, pyruvate kinase, ions, and other factors and co-factors. Alternatively, in vitro translation of CAR mRNA molecules can be performed using supplemented eukaryotic extract systems, such as supplemented reticulocyte lysate, or supplemented wheat germ extract.

### EXAMPLES

The invention will now be further described by way of examples, which are meant to serve to assist one of ordinary skill in the art in carrying out the invention and are not intended in any way to limit the scope of the invention.

The sequences of the different CAR proteins described in the examples include SEQ ID No. 57, SEQ ID No. 58, SEQ ID No. 59, SEQ ID No. 60, SEQ ID No. 61, SEQ ID No. 62, SEQ ID No. 63 and SEQ ID No. 64.

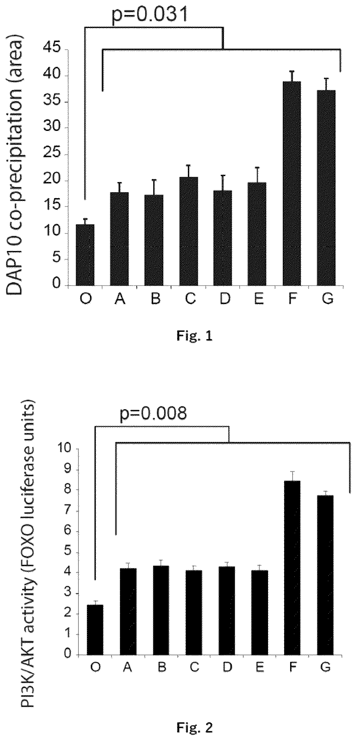

SEQ ID No. 57 is a synthetic protein, namely CAR, resulting from the link of SEQ ID No. 65 (as a signal peptide), SEQ ID No. 4 (as an antigen-binding domain), SEQ ID No. 12 (as a CD68 ectodomain spacer), SEQ ID No. 13 (as a native GLUT4 transmembrane domain), and SEQ ID No. 48 (as a synthetic GLUT4 endodomain). On figures 1 to 3, SEQ ID No. 57 is represented by the reference "O".

SEQ ID No. 58 is a similar synthetic combination as of SEQ ID No. 57, but with SEQ ID No. 17 instead of the native GLUT4 transmembrane domain. On figures 1 to 3, SEQ ID No. 58 is represented by the reference "A".

SEQ ID No. 59 is a similar synthetic combination as of SEQ ID No. 57, but with SEQ ID No. 18 instead of the native GLUT4 transmembrane domain. On figures 1 to 3, SEQ ID No. 59 is represented by the reference "B".

SEQ ID No. 60 is a similar synthetic combination as of SEQ ID No. 57, but with SEQ ID No. 37 instead of the native GLUT4 transmembrane domain. On figures 1 to 3, SEQ ID No. 60 is represented by the reference "C".

SEQ ID No. 61 is a similar synthetic combination as of SEQ ID No. 57, but with SEQ ID No. 40 instead of the native GLUT4 transmembrane domain. On figures 1 to 3, SEQ ID No. 61 is represented by the reference "D".

SEQ ID No. 62 is a similar synthetic combination as of SEQ ID No. 57, but with SEQ ID No. 41 instead of the native GLUT4 transmembrane domain. On figures 1 to 3, SEQ ID No. 62 is represented by the reference "E".

SEQ ID No. 63 is a similar synthetic combination as of SEQ ID No. 57, but with SEQ ID No. 38 instead of the native GLUT4 transmembrane domain. On figures 1 to 3, SEQ ID No. 63 is represented by the reference "F".

SEQ ID No. 64 is a similar synthetic combination as of SEQ ID No. 57, but with SEQ ID No. 39 instead of the native GLUT4 transmembrane domain. On figures 1 to 3, SEQ ID No. 64 is represented by the reference "G".

### Example 1 - Enhanced recruitment of signaling partners by synthetic GLUT4 transmembrane domain containing CAR

The CAR proteins were cloned into a lentivirus vector (such as those described in Guenechea G et al (Mol Ther. 2000 Jun;1(6):566-73)) and transduced into HYG1 cells. CAR protein was precipitated with MYC tag (Kimple ME et al (Curr Protoc Protein Sci. 2013 Sep 24;73:9.9.1-9.9.23) and Terpe K. (Appl Microbiol Biotechnol. 2003 Jan;60(5):523-33)) from CAR-containing HYG1 cells. Co-immunoprecipitated protein was separated through western blot and then was stained through immunoblotting assay against DAP10 and quantified. Immunoblotting assay quantification of the area units show us that DAP10 co-precipitate more in the HYG1 cells containing the CAR with one synthetic GLUT4 transmembrane domain selected among SEQ ID No. 17, SEQ ID No. 18 SEQ ID No. 37 SEQ ID No. 40, SEQ ID No. 41, SEQ ID No. 38 an SEQ ID No. 39 than the native sequence counterpart GLUT4 transmembrane of SEQ ID No 13 (Figure 1), i.e., the recruitment of DAP10 signaling protein is enhanced.

**Example 2** - Demonstration that NK cells expressing CAR containing synthetic GLUT4 transmembrane domain with enhanced recruitment capacity of signaling partners, such as DAP10, undergo stronger activation of PI3K/AKT pathway.

The CAR proteins were cloned into a lentivirus vector and transduced into HYG1 cells. A FOXO-luciferase reporter (as that described in Aldonza MBD et al (Sci Adv. 2020 Feb 7;6(6):eaav7416)) was transduced into the CAR containing HYG1 cells. Quantification of PI3K/AKT pathway was performed after co-culture exposure of HYG1 cells to CD19 positive malignant cells. The results demonstrate that NK cells containing CAR with one synthetic GLUT4 transmembrane domain selected among SEQ ID No. 17, SEQ ID No. 18 SEQ ID No. 37 SEQ ID No. 40, SEQ ID No. 41, SEQ ID No. 38 an SEQ ID No. 39 have higher activation of PI3K/AKT pathway than the native sequence counterpart GLUT4 transmembrane of SEQ ID No. 13 (Figure 2).

**Example 3** - Demonstration of anti-tumorigenic properties of NK cells expressing CAR containing synthetic GLUT4 transmembrane domain with enhanced recruitment capacity of signaling partners, as DAP10, which provide higher clearing efficiency of CD19 positive malignant cells.

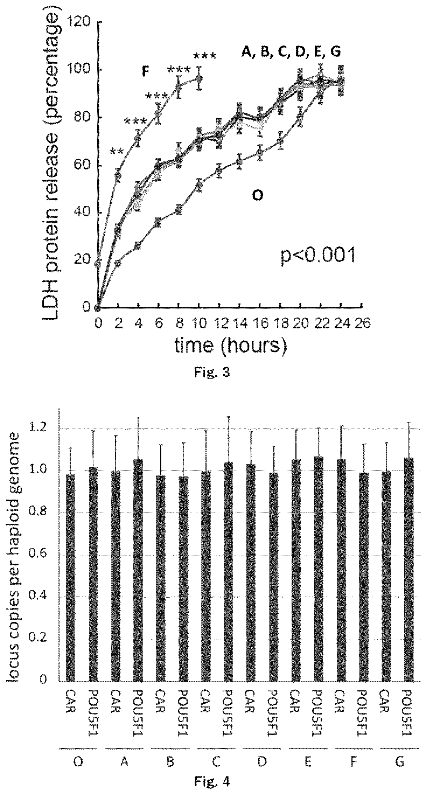

The CAR proteins were cloned into a lentivirus vector (such as those described in Guenechea et al. (cfr. supra)) and transduced into HYG1 cells. HYG1 cells containing CAR with transmembrane domains of SEQ ID No. 17, SEQ ID No. 18 SEQ ID No. 37 SEQ ID No. 40, SEQ ID No. 41, SEQ ID No. 38 an SEQ ID No. 39 were cultured with CD19 expressing malignant cell SU-DHL-4. Quantification of lactate dehydrogenase (LDH) (such as an assay described in Cox MC et al. (Sci Rep. 2021 Sep 17;11(1):18571)) demonstrates that CD19-expressing cells undergo cell death. The results demonstrate that NK cells containing the CAR with one synthetic GLUT4 transmembrane domain selected among SEQ ID No. 17, SEQ ID No. 18 SEQ ID No. 37 SEQ ID No. 40, SEQ ID No. 41, SEQ ID No. 38 an SEQ ID No. 39 are clearly more efficient at inducing cell death on CD19-positive malignant cells than the native sequence counterpart GLUT4 transmembrane of SEQ ID No 13 (Figure 3).

Those experiments also show that among the advantageous CARs with GLUT4 transmembrane domain as described herein, the CAR with GLUT4 transmembrane domain SEQ ID No. 38 or SEQ ID No. 39 is the most efficient in binding signaling partners, eliciting pathway activation and clearing a malignant model of CD19 positive cells.

**Example 4** - Double copy integration of CAR containing synthetic GLUT4 transmembrane domain with enhanced recruitment capacity of signalling partners, such as DAP10, into induced pluripotent stem cells.

The CAR genes were cloned into a donor vector and transfected into iPSC (in a similar manner to Zhang J, Hu Y, Yang J, et al. Development and clinical evaluation of non-viral genome specific targeted CART cells in relapsed/refractory B-cell non-Hodgkin lymphoma. medRxiv; 2020. DOl: 10.1101/2020.09.22.20199786.). The genetic dose of CAR gene was determined by quantitative real time PCR, using absolute quantification, and its dose compared to the copy number of other loci (POU5F1 and FBXO1). These quantifications demonstrate CAR gene with one synthetic GLUT4 transmembrane domain sequence selected among SEQ ID No. 17, SEQ ID No. 18 SEQ ID No. 37 SEQ ID No. 40, SEQ ID No. 41, SEQ ID No. 38 an SEQ ID No. 39 integrated in homozygous manner, with two copies, into clonal iPSC (Figure 4).

### Example 5 - Enhanced recruitment of signalling partners by synthetic GLUT4 transmembrane domain containing CAR.

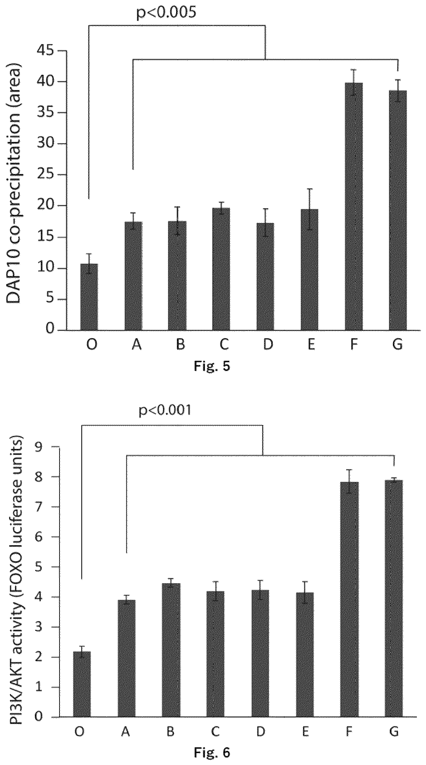

The CAR proteins were cloned into a donor vector and transfected into iPSC (Example 4). The iPSC were differentiated into NK cells. CAR protein was precipitated with MYC tag from CAR containing iPSC-derived NK cells. Co-immunoprecipitated protein was stained against DAP10 and quantified. These quantifications show that DAP10 co-precipitates more in the iPSC-derived NK cells containing the CAR with one synthetic transmembrane domain selected among SEQ ID No. SEQ ID No. 17, SEQ ID No. 18 SEQ ID No. 37 SEQ ID No. 40, SEQ ID No. 41, SEQ ID No. 38 an SEQ ID No. 39 than with the native sequence counterpart GLUT4 transmembrane of SEQ ID No. 13 (Figure 5).

**Example 6** - Demonstration that iPSC-derived NK cells expressing CAR containing synthetic GLUT4 transmembrane domain with enhanced recruitment capacity of signalling partners, such as DAP10, undergo stronger activation of PI3K/AKT pathway.

The CAR proteins were cloned into a donor vector and transfected into iPSC. A FOXO-luciferase reporter was transduced into the CAR containing iPSC. The iPSCs were differentiated into NK cells. Quantification of PI3K/AKT pathway was performed after exposure of iPSC-derived NK cells to CD19 positive malignant cells. The results demonstrate that iPSC-derived NK cells containing CAR with one synthetic GLUT4 transmembrane domain selected among SEQ ID No. 17, SEQ ID No. 18 SEQ ID No. 37 SEQ ID No. 40, SEQ ID No. 41, SEQ ID No. 38 an SEQ ID No. 39 have higher activation of PI3K/AKT pathway than with the native sequence counterpart GLUT4 transmembrane transmembrane of SEQ ID No 13 (Figure 6).

**Example 7** - Demonstration of anti-tumorigenic properties of iPSC-derived NK cells expressing CAR containing synthetic GLUT4 transmembrane domain with enhanced recruitment capacity of signalling partners, as DAP10, which provide higher clearing efficiency of CD19 positive malignant cells.

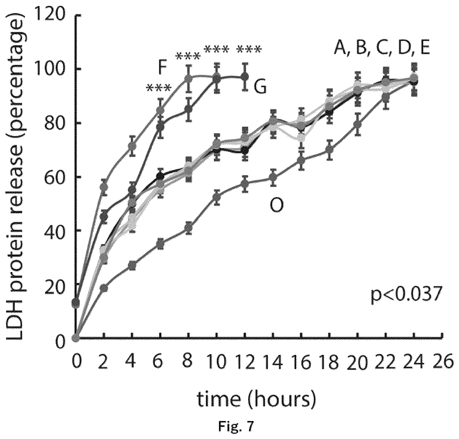

The CAR proteins were cloned into a donor vector and transfected into iPSC. The iPSCs were differentiated into NK cells. Then, these iPSC-derived NK cells containing CAR with GLUT4 transmembrane domains of SEQ ID No. 17, SEQ ID No. 18 SEQ ID No. 37 SEQ ID No. 40, SEQ ID No. 41, SEQ ID No. 38 an SEQ ID No. 39 were cultured with CD19 expressing malignant cell SU-DHL-4. Quantification of lactate dehydrogenase (LDH) demonstrates that CD19-expressing cells undergo cell death. The results demonstrate that iPSC-derived NK cells containing the CAR with one synthetic GLUT4 transmembrane domain selected among SEQ ID No. 17, SEQ ID No. 18 SEQ ID No. 37 SEQ ID No. 40, SEQ ID No. 41, SEQ ID No. 38 an SEQ ID No. 39 are clearly more efficient at inducing cell death on CD19-positive malignant cells than with the native sequence counterpart GLUT4 transmembrane transmembrane of SEQ ID No 13 (Figure 7).

Those experiments also show that the CAR with transmembrane domain SEQ ID No. 38 or SEQ ID No. 39 is more efficient in binding signaling partners, eliciting pathway activation and clearing a malignant model of CD19 positive cells by NK cells derived from iPSC.

## Claims

1. A chimeric antigen receptor (CAR), comprising:
a. an antigen-binding domain, wherein the antigen-binding domain is a differentially expressed marker-binding domain, and comprises a single chain variable fragment (scFv);
b. an ectodomain spacer;
c. a synthetic GLUT4 transmembrane peptide composed of 19 to 23 amino acids; and
d. an endodomain;
wherein in the at least one of the amino acids from 1 to 13 of said synthetic GLUT4 transmembrane peptide is selected from arginine, histidine or lysine.

2. The CAR according to claim 1, wherein
a. the at least one of the amino acids from 1 to 13 of the synthetic GLUT4 transmembrane peptide is arginine, or
b. at least two of the amino acids from 1 to 13 of the synthetic GLUT4 transmembrane peptide are arginines.

3. The CAR according to any one of the preceding claims, wherein the endodomain is a GLUT4 endodomain; preferably, wherein the GLUT4 endodomain encompasses at least one di-leucine motif.

4. The CAR according to any one of the preceding claims, wherein the ectodomain spacer comprises at least one CD68 protein ectodomain, and/or wherein the antigen-binding domain is a CD19-binding domain.

5. The CAR according to any one of the preceding claims, **characterized in that** the synthetic GLUT4 transmembrane peptide comprises a sequence selected from SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 29, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, or a variant thereof having at least 80% sequence identity.

6. The CAR according to any one of claims 3 to 5, **characterized in that** the GLUT4 endodomain comprises a sequence selected from SEQ ID No. 46, SEQ ID No. 47, SEQ ID No. 48, or a variant thereof having at least 80% sequence identity.

7. The CAR according to any one of claims 4 to 6, **characterized in that** the CD68 ectodomain spacer comprises a sequence selected from SEQ ID No. 6, SEQ ID No. 7, SEQ ID No. 8, SEQ ID No. 9, SEQ ID No. 10, SEQ ID No. 11, SEQ ID No. 12, or a variant thereof having at least 80% sequence identity.

8. The CAR according to any one of the preceding claims, wherein:
a. the single chain variable fragment (scFv) comprises a heavy chain variable region (VH), having a sequence SEQ ID No. 2, and/or a light chain variable region (VL), having a sequence SEQ ID No. 3; and/or
b. the single chain variable fragment (scFv) comprises a heavy chain variable region (VH) and a light chain variable region (VL), and the regions are oriented in the direction VH-VL or VL-VH.

9. The CAR according to any one of the preceding claims, **characterized in that** it comprises one of the following sequences as indicated in SEQ ID No. 49, SEQ ID No. 50, SEQ ID No. 51, SEQ ID No. 52, SEQ ID No. 53, SEQ ID No. 54, SEQ ID No. 55, SEQ ID No. 56, SEQ ID No. 57, SEQ ID No. 58, SEQ ID No. 59, SEQ ID No. 60, SEQ ID No. 61, , SEQ ID No. 62, SEQ ID No. 65, or SEQ ID No. 64, or a variant thereof having at least 80% sequence identity.

10. A nucleic acid sequence which encodes a CAR according to any one of claims 1 to 9.

11. A vector which comprises a nucleic acid sequence according to claim 10.

12. A cell which comprises a CAR according to any of claims 1 to 9 and/or a nucleic acid sequence according to claim 10; preferably, wherein the cell is a natural killer cell, an induced natural killer cell, a hematopoietic stem cell, a hematopoietic stem cell like-cell, an induced pluripotent stem cell, or an induced pluripotent stem cells-derived natural killer cell; more preferably, wherein the cell is an induced pluripotent stem cells-derived natural killer cell.

13. A pharmaceutical composition which comprises a cell according to claim 12 and a pharmaceutically acceptable carrier, diluent and/or excipient.

14. A cell according to claim 12 or a pharmaceutical composition according to claim 13 for use in treating cancer; preferably, for use in a treatment of CD19 positive B cell malignancy.

15. A method for making a cell according to claim 12, wherein the method comprises a step of transducing or transfecting a cell with a vector according to claim 11; preferably, wherein the step of transducing or transfecting a cell with said vector is performed ex *vivo.*
